# EUROPÄISCHE PATENTSCHRIFT

(11) **EP 1 898 851 B1**
(45) Veröffentlichungstag und Bekanntmachung des Hinweises auf die Patenterteilung: **25.08.2010**
(21) Anmeldenummer: 06754041.9
(22) Anmeldetag: 01.06.2006
(51) Int. Cl.: A61F 7/00

(54) **VORRICHTUNG ZUM ANLEGEN ODER VERABREICHEN VON PACKUNGEN**
DEVICE FOR APPLYING OR ADMINISTERING COMPRESSES
DISPOSITIF POUR APPLIQUER OU ADMINISTRER DES ENVELOPPEMENTS

(30) Priorität: 02.06.2005 DE 102005025471
(43) Veröffentlichungstag der Anmeldung: 19.03.2008
(73) Patentinhaber: Haslauer, Paul, A-5020 Salzburg (AT)
(72) Erfinder: Haslauer, Paul, A-5020 Salzburg (AT)
(74) Vertreter: Flach, Dieter Rolf Paul
(86) Internationale Anmeldenummer: PCT/EP2006/005234
(87) Internationale Veröffentlichungsnummer: WO 2006/128703

(56) Entgegenhaltungen:
- EP-A- 0 552 397
- WO-A-92/21306
- US-A- 1 653 901
- US-A- 4 657 531

## Beschreibung

Die Erfindung betrifft eine Vorrichtung zum Anlegen oder Verabreichen von Packungen.

Wärmeanwendungen zählen zu sehr alten Therapieformen. Dabei sind prinzipiell zwei grundsätzlich verschiedene Methoden zu unterscheiden, nämlich einerseits die Verabreichung von heißen Bädern und andererseits die lokal beschränkte Anwendung von heißen Packungen.

Unter Wärmepackungen versteht man in Fachkreisen eine örtliche Überwärmebehandlung in der Regel unter Verwendung von Peloiden, Schlämmen, Kräutermischungen oder auch flüssigen oder schmierfähigen Lotionen oder sonstigen Wirkstoffzubereitungen oder -lösungen, verbunden mit der Möglichkeit, weitaus höhere Temperaturen als bei den oben erwähnten Bädern anzuwenden.

Wärmepackungen dienen vor allem zur lokalen Überwärmung nur bestimmter Körperregionen. Da die Wärme vor allem nur in bestimmten Bereichen und/oder nur zeitlich beschränkt einwirken soll, können höhere Temperaturen angewendet werden, als üblicherweise bei Bädern. Problematisch ist allerdings, dass die Packungen zunächst aufgeheizt werden müssen, um sie dann bei der Behandlung an bestimmten Körperstellen anzulegen. Von daher lässt sich mit derartigen Packungen nur ein über die Zeit abnehmendes Temperaturprofil erzielen.

Eine wesentliche Verbesserung ist beispielsweise auch schon unter Verwendung einer Packung gemäß der DE-PS 2 611 928 möglich geworden, wobei gemäß dieser Vorveröffentlichung vorgeschlagen worden ist, zwei Beutel in Anlage zu bringen, nämlich eine Heißpackung, die beispielsweise mit Peloid gefüllt ist, und einen zweiten darauf auflegbaren Beutel, der als Wärmespeicher gilt.

Weitere Verbesserungen sind schließlich auch gemäß der europäischen Patentschrift EP 0 144 571 B1 dadurch erzielt worden, dass Packungen an den gewünschten Körperstellen aufgelegt und die verbleibenden Körperstellen isoliert oder zumindest mit die Wärmeübertragung verringernden Materialien geschützt werden, um dann anschließend die betreffende Person mit den angelegten Packungen und den dazwischen befindlichen Isoliermaterialien auf einer Andrückfolie liegend bevorzugt in ein mit Wärmemedium befülltest Becken herabzulassen. Auf der Unterseite der Andrückfolie befindet sich z.B. aufgeheiztes Wasser oder ein sonstiges geeignetes Medium, welches nunmehr als Wärmequelle zur ständigen Zuführung und Erwärmung der Packungen dient.

Die erwähnten Packungen, beispielsweise unter Verwendung von Schlämmen, Peloiden, Kräutern, Wirkstoffen, Zubereitungen und dergleichen werden heute häufig nicht wie bisher üblich nur einfach an bestimmten Körperstellen angelegt, sondern diese Packungen werden verwendet, um sie in Massagebewegungsabläufe einzubauen. Dadurch soll ein noch besseres Wohlbefinden erzielt werden. Dabei werden die Packungen von einem die Anwendung verabreichenden Betreuer ergriffen und beispielsweise in kreisenden Bewegungen über eine auf einer Liege befindlichen Person hinweggeführt. Dabei kann die Geschwindigkeit der Bewegung ständig den Erfordernissen folgend variiert und auch der Anpressdruck verändert werden. Ein die Anwendung verabreichender Betreuer oder Masseur kann dabei beispielsweise seine linke Hand diagnostizierend über den Körper einer Person hinwegbewegen, um festzustellen, wo beispielsweise verhärtete Muskelpartien aufzufinden sind, um anschließend die in der anderen Hand gehaltene und aufgewärmte Packung vor allem über diese Körperstellen massierend hinwegzubewegen.

Ferner ist es bekannt, auch vorgewärmte Steine zu verwenden und auf einer mit Öl und Wasser vorbehandelten Haut massierende Bewegungsabläufe durchzuführen. Wenn der verwendete Stein schon etwas abgekühlt ist, wird er oftmals an bestimmten Körperstellen angelegt und dort liegengelassen. Dies gestaltet sich jedoch als schwierig. Um eine noch fühlbare Restwärme vorhalten zu können, muss der Stein relativ hoch temperiert angelegt werden. Gerade noch unterhalb jener Temperatur, bei der eine Berührung mit der Haut irreversible Schäden verursachen würde. Oft wird diese punktuelle Wärmezufuhr als unangenehm empfunden.

Eine gattungsbildende Vorrichtung, die den nächsten stand der Technik bildet, ist beispielsweise aus der WO 92/21306 A1 bekannt geworden. Es handelt sich dabei um ein handgehaltenes Dampfgerät mit einem Poliermassagekopf, der durch ein im Gehäuse integriertes Antriebsmittel in Rotation versetzbar ist. An dem drehenden Poliermassagekopf ist ein poröser Polsterbelag abnehmbar befestigt, der zusammen mit dem Dampfgenerator die Hautoberfläche gleichzeitig behandelt, während der Dampf von dem Dampferzeuger den Hauptoberflächen zugeführt wird.

Der Polsterbelag besteht aus einem natürlichen Fasermaterial oder aus einem Fadenfasermaterial oder aus einem abreibenden Material oder aus einem weichen saugfähigen Material. Gemäß der vorstehend genannten Vorveröffentlichung können auf dem Polsterbelag Medikationsmittel aufgebracht werden. Alternativ dazu ist es auch möglich, dass der Dampferzeuger einen Vorrat an Wasser und Medikationsmittel enthält, um den Hautoberflächen mit Medikationsmittel versetzten Dampf zuzuführen.

Gemäß einer Ausführungsvariante kann dabei der Polsterbelag unter Verwendung von Haken und Ösen oder unter Verwendung eines geschlitzten Ringes abnehmbar am Massagekopf befestigt sein.

Der drehende abnehmbare Massagekopf kann unterschiedlichste Formen aufweisen. So kann seine am Gesicht anzulegende Behandlungsseite kegelförmig oder pilz- oder konvexförmig gestaltet sein. Möglich ist auch eine Ausgestaltung des Massagekopfes nach Art einer flachen Scheibe, die geringstfügig konkav gestaltet ist, wobei alle Ausführungsformen des so gebildeten Massagekopfs zentral von innen bis nach außen zum Rand verlaufend mit Austrittsöffnungen versehen sind, durch welche der zugehörige Dampf hindurchströmen kann.

Eine Vorrichtung zur Behandlung der Haut ist beispielsweise auch aus der US 4, 657, 531 bekannt geworden. Bei diesem vorbekannten Gerät ist lediglich eine Heizeinrichtung vorgesehen, die behandlungsseitig mit einer Heizplatte abgeschlossen ist. Oberhalb der Heizplatte kann an dem Gerät ein Gewebe-Pad aufgelegt sein, welches beispielsweise mit Kräutern und dergleichen befüllt sein kann. Es handelt sich dabei um eine Art Packung. Am Behandlungskopf kann ein Gewebeüberzug verwendet werden, der über die Packung gezogen und dann an dem Gerät befestigt wird. Darüber hinaus kann auch die Heizplatte selbst von dem Rest des Gerätes entfernt werden. Damit das mit der Haut in Berührung kommende und die Packung zurückhaltende Gewebe entsprechend festsitzt, wird es über Federeinrichtungen an der elektrischen Heizeinrichtung außenliegend befestigt.

Aufgabe der vorliegenden Erfindung ist es von daher, ausgehend vom gattungsbildenden Stand der Technik eine verbesserte Vorrichtung zum Anlegen sowie zur Verabreichung einer Packung sowie eine zugehörige Packungshalterung zu schaffen.

Die Aufgabe wird erfindungsgemäß bezüglich der Vorrichtung entsprechend den im Anspruch 1 angegebenen Merkmalen gelöst. Vorteilhafte Ausgestaltungen der Erfindung sind in den Unteransprüchen angegeben.

Durch die vorliegende Erfindung wird insbesondere für die Verabreichung entsprechender Packungen durch geschultes Personal, insbesondere durch Masseure, eine deutliche Verbesserung gegenüber herkömmlichen Lösungen erzielt.

Mit der erfindungsgemäßen Vorrichtung ist es möglich, eine Packung unter Durchführung von Massagebewegungen während der Bewegung laufend an den Bedarf bzw. an bestehende Bedürfnisse anzupassen. Dies gilt vor allem hinsichtlich der Temperatur und der Wärmeübertragung. Denn erfindungsgemäß ist nunmehr vorgesehen, dass die verabreichte Packung auf einem bestimmten Temperaturniveau gehalten, bei Bedarf sogar weiter aufgeheizt, oder in einer bevorzugten Ausführungsform sogar in einem bestimmten Zeitprofil gekühlt werden kann, ohne dass die Verabreichung und die Massagebewegung unterbrochen werden müssen. Insoweit umfasst die erfindungsgemäße Vorrichtung eine Temperatursteuerungseinrichtung, um ein vorstehend erläutertes Temperaturprofil beliebig einstellen zu können. Zudem wird erfindungsgemäß eine Haltevorrichtung für eine entsprechende Packung vorgeschlagen, die bevorzugt mit einem multifunktionalen Haltegriff zusammenwirkt. Über den Haltegriff kann beispielsweise ein Masseur die Packung unter Erzielung der gewünschten Massagebewegung oder auch unter teilweisem Innehalten an bestimmten Körperstellen gezielt einsetzen und dabei je nach Bedarf durch die Betätigungs- und/oder Auslöseeinrichtung die Temperatur auf ein bestimmtes Niveau erhöhen, dabei die Temperatur zeitlich länger oder kurzfristiger erhöhen, teilweise wieder absenken etc.

Bevorzugt kann dies durch Zuführung eines gasförmigen Mediums, insbesondere durch Luft oder Dampf erfolgen. Dazu ist die Halte- und Betätigungseinrichtung über eine Schlauchverbindung oder schlauchartige Verbindung mit einer Aufheizvorrichtung zur Erzeugung von Heißluft oder heißem Dampf verbunden.

Auch die Temperatursteuerung kann bevorzugt direkt von der Halte- und/oder Betätigungseinrichtung für die Packung ausgelöst und gesteuert werden.

Bevorzugt kann die schlauchförmige Einrichtung auch aus zwei Schläuchen oder einem Schlauch mit einem Zweikammersystem etc. bestehen, um wechselweise bei Bedarf eventuell auch ergänzend Warmwasser oder ein sonstiges fließfähiges Medium oder bestimmte Wirkstoffe und Extrakte separat gesteuert zuzuführen.

Schließlich ist in einer besonders bevorzugten Ausführungsform der Erfindung auch vorgesehen, die Temperaturregelung so zu gestalten, dass zeitlich gesteuert eventuell auch kühles Medium zugeführt werden kann. Eventuell kann in einem schnellen Rhythmuswechsel eine kurzzeitige Überhitzung an bestimmten Körperstellen durchgeführt werden, denen eine kurzzeitige Unterkühlung folgt.

Auch hier könnte es gegebenenfalls sinnvoll sein, zumindest zwei oder gegebenenfalls noch mehr Schläuche oder Schlauchsysteme zu verwenden, wobei in den einzelnen Schläuchen oder Kammern dieses Systems unterschiedliche Medien oder unterschiedlich aufgeheizte Medien direkt am zugehörigen Haltegriff, also direkt kurz vor der Packung bereitstehen, so dass gezielt ein schneller Temperaturwechsel an bestimmten Körperstellen durchgeführt werden kann, also insbesondere kurzzeitig eine Überwärmung bzw. Abkühlung.

Ergänzend kann die Temperatursteuerung beispielsweise aber auch über eine elektrische Aufheizeinrichtung in oder an der Packung realisiert werden.

Wird die Steuerung nicht über die entsprechende Bediener-Schaltung am Haltegriff oder an der Haltegriffeinrichtung ausgelöst und umgesetzt, so kann es sich ebenfalls als günstig erweisen, beispielsweise am Haltegriff einen Auslöser oder dergleichen zu betätigen, der beispielsweise mit einem Ventil, einer Ventilsteuerung, einer Steuerungseinrichtung zum Aufheizen, Abkühlen etc. verbunden ist (beispielsweise über Funk, Infrarot oder beispielsweise über eine Niederspannungs-Leitung), um dann bei einem entfernt angeordneten und über die Schlauchverbindung mit dem Haltegriff verbundenen Wärme- und/oder Kühlgenerator die entsprechende Ansteuerung durchzuführen. Dies kann über mechanisch betätigbare Steuerungsleitungen oder bevorzugt im Niederspannungsbereich mittels elektrischen Leitungen erzielt werden. Wie erwähnt, sind auch ferngesteuerte Betätigungen denkbar. Mittels einer derartigen Einrichtung ist es auch möglich, die zugeführte Wärme oder das zugeführte gekühlte Medium entsprechend den Bedürfnissen und Anforderungen zu dosieren, vor allem auch bei den durchgeführten Massagebewegungen der Packung. Dies kann beispielsweise durch entsprechende Ansteuerung von vorgesehenen Potentiometern, Durchflussreglern, Ventilen etc. bewerkstelligt werden.

Der Befestigungskopf selbst umfasst eine Aufnahme mit einem Aufnahmeraum und einer zugehörigen Öffnung. Hierüber kann eine an dem Befestigungskopf anzubringende Packung in den Aufnahmeraum eingeführt werden. Der Aufnahmeraum selbst ist größer als der entsprechende Temperaturverteiler gestaltet, durch den hindurch das zuzuführende Behandlungsmedium in den Aufnahmeraum und damit durch die Packung hindurch strömen kann. Dadurch wird gewährleistet, dass die Packung durch Zuführen entsprechender fließfähiger Medien so aufbereitet und die darin befindlichen Wirkstoffe aufgeschüttelt werden, dass ein optimaler Effekt beim Einsatz des erfindungsgemäßen Gerätes erzielt werden kann.

Als Packung kommen alle nur erdenklichen Packungen in Frage. Diese können bevorzugt mit bestimmten Medien und/ oder Wirkstoffen etc. befüllt sein, beispielsweise unter Verwendung von Kräutern, Wirkstoffzubereitung, Peloiden und dergleichen. Die Packung weist bevorzugt eine zumindest wirkstoffdurchlässige Oberfläche oder Umhüllung auf.

In einer besonders bevorzugten Ausführungsform wird eine derartige Packung in einer weiteren Packungsumhüllung integriert, in welche der erfindungsgemäße Halte- und Befestigungskopf der Vorrichtung mit befestigt werden kann. Die Packung bzw. deren zusätzliche Umhüllung ist dabei so aufgebaut, dass der Haltegriff aus isolierendem Material an der Packung rasch angebracht und abgenommen werden kann.

Bevorzugt ist der erfindungsgemäße Haltegriff der Halteeinrichtung so ausgeführt, dass er beispielsweise mit einem Hand- oder Daumengriff oder dergleichen oder durch eine Drehbewegung etc. schnell und unkompliziert eine Spannung auf die erwähnte äußere Umhüllüng der Packung ausübt und diese nachspannt. Auch die sonstigen Betätigungseinrichtungen zur Temperatursteuerung etc. sind leicht bedienbar vorgesehen und angebracht.

Bevorzugt ist die Halteeinrichtung bzw. der Haltegriff und ein zugehöriger Verbindungsschlauch drucklos gehalten, um eine rasche An- und Abkopplung zu erzielen. Der Haltekopf ist dabei mit einem geeigneten Austrittskopf zur Zuführung von Luft, Heißluft, Dampf, gegebenenfalls auch zur Zuführung von flüssigem Medium wie Warmwasser, Heißwasser etc. geeignet gestaltet, kann also insoweit kugelförmig, teil- oder halbkugelförmig, pilzförmig oder dergleichen geformt sein und dazu einen größeren Austrittskopf (vorzugsweise mit einer Vielzahl von Austrittsöffnungen) aufweisen, als ein darunter befindlicher mit kleinerem Durchmesser gestalteter Hals. Dadurch kann der Befestigungs- und Austrittskopf besonders günstig von einer Packung umkleidet und vor allem unter Verwendung der erwähnten zusätzlichen Außenhülle mit einer so gebildeten Packung fest verbunden werden, beispielsweise durch eine geeignete Greifer- und/oder Krallen-Spannvorrichtung durch eine sichere Verbindung mit der Packung herstellbar und dabei auch je nach Bedarf eine schnelle Nachspannung durchführbar ist, was insofern auch von Bedeutung ist oder sein kann, da das Volumen der Packung während der Behandlung abnimmt oder abnehmen kann.

Schließlich kann in einer bevorzugten Ausführungsform noch kurz vor der Packung, vorzugsweise im Haltegriff oder davor - also allgemein zwischen der Aufheizvorrichtung (Verdampfer) und der Packung - eine ergänzende Wirkstoffzufuhr vorgesehen sein, die beispielsweise eine Einlagevorrichtung für echte Kräuter umfasst. Darüber können an die Umgebung oder über das Schlauchsystem weiter in Richtung Packung auch noch bestimmte Wirkstoffe, Kräuteressenzen etc. zugeführt werden.

Die Erfindung wird nachfolgend anhand von Zeichnungen näher erläutert. Dabei zeigen im Einzelnen:
- Figur 1:: eine schematische perspektivische Darstel- lung einer eher rechteckförmigen oder qua- dratischen Packung;
- Figur 2:: eine schematische perspektivische Darstel- lung einer scheibenförmigen Packung;
- Figur 3:: eine schematische perspektivische Darstel- lung einer perforierten Außenumhüllung zur Aufnahme einer Packung;
- Figur 4:: eine schematische Querschnittsdarstellung durch die in Figur 3 wiedergegebene Außen- umhüllung mit entsprechend eingesetzter Packung;
- Figur 5:: eine schematische Seitendarstellung durch eine Vorrichtung (nicht zur Erfindung gehörend) mit auf- gesetzter Packung;
- Figur 6:: eine schematische axiale Querschnittsdar- Stellung aurcn ein erfindungsgemäßes abgewandeltes Ausfüh- rungsbeispiel eines Befestigungskopfes mit einem Temperaturverteiler; und
- Figur 7:: eine schematische perspektivische Darstel- lung einer spezifisch angepassten Packung.

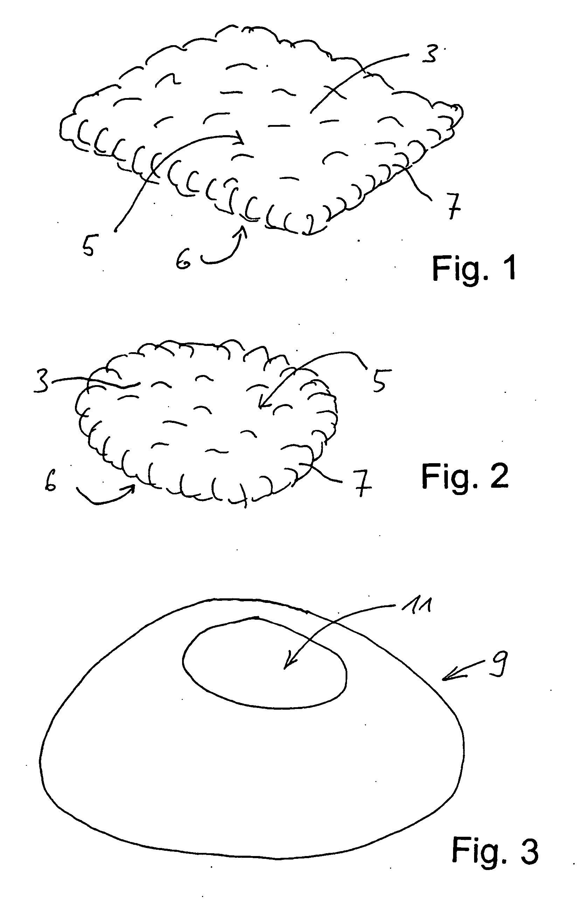

In Figur 1 ist in schematischer Darstellung ein Beispiel für eine Packung 1 gezeigt, die kissen- oder padförmig gestaltet sein kann. Die Außenabmessungen in Draufsicht können beispielsweise quadratisch oder rechteckförmig oder an diese Formen angenähert sein. Die Dicke sollte in der Regel nicht zu stark bemessen sein, beispielsweise zwischen 1, 2 oder 3 mm Minimum und 2, 3, 4 oder 5 cm Maximum liegen. Eine Außenumhüllung 3 der Packung ist glas-, d.h. luft-, dampf- und/oder wasserdurchlässig gestaltet, d.h. sie ist ganz oder überwiegend perforiert gestaltet, vor allem in ihrem gegenüberliegenden groß dimensionierten Ober- und Unterseitenbereich 5, 6.

Ansonsten können die im Rahmen der Erfindung verwendeten Packungen weitgehend beliebige Formen aufweisen, in Draufsicht n-polygonal gestaltet sein und dergleichen.

Anhand von Figur 2 ist nur angedeutet, dass auch eine in Draufsicht eher runde Gestaltung ebenso möglich ist.

Derartige Packungen 1 werden zur Verwendung im Zusammenhang mit der erfindungsgemäßen Vorrichtung bevorzugt in einer Außenumhüllung 9 gegeben (Figur 3), die ebenfalls wiederum ganz oder in weiten Bereichen Luft-, gas-, dampf- und/oder wasserdurchlässig ist. Die Außenumhüllung 9 kann beispielsweise ebenso wie die Packung selbst aus einem Fließ oder allen sonstigen geeigneten Materialien gebildet sein. Diese Außenumhüllung 9 sollte aber ebenfalls wieder gas-, d.h. insbesondere luft-, dampf- und/oder wasserdurchlässig gestaltet sein, kann also von daher mit einer Perforation versehen sein, wenn das verwendete Material selbst beispielsweise nicht gas- und/oder wasserdurchlässig sein sollte. Die Außenumhüllung 9 kann in ihrer maximalen Umfangserstreckung beutelförmig gestaltet sein, beispielsweise kugelförmig oder eher diskusförmig etc. Bevorzugt weist die Außenumhüllung 9 eine ausreichend groß dimensionierte Einführöffnung 11 auf, in welche die entsprechend verwendeten Packungen 1 eingeführt werden können. Genauso ist es möglich, eine Außenumhüllung oder Außenhülle 9 zu verwenden, welche aus einem eingeschlagenen Tuch besteht, (welches also von der dem Körper zugewandt liegenden Seite ausgehend zur rückwärtigen Seite zusammengebunden wird) wobei die Ecken jeweils nach oben gezogen und verspannt werden. Dadurch kann ein zentraler freibleibender Raum gebildet werden, der an seiner rückwärtigen Seite eine entsprechende dimensionierte Einführöffnung 11 aufweist, auf deren Bedeutung später eingegangen wird.

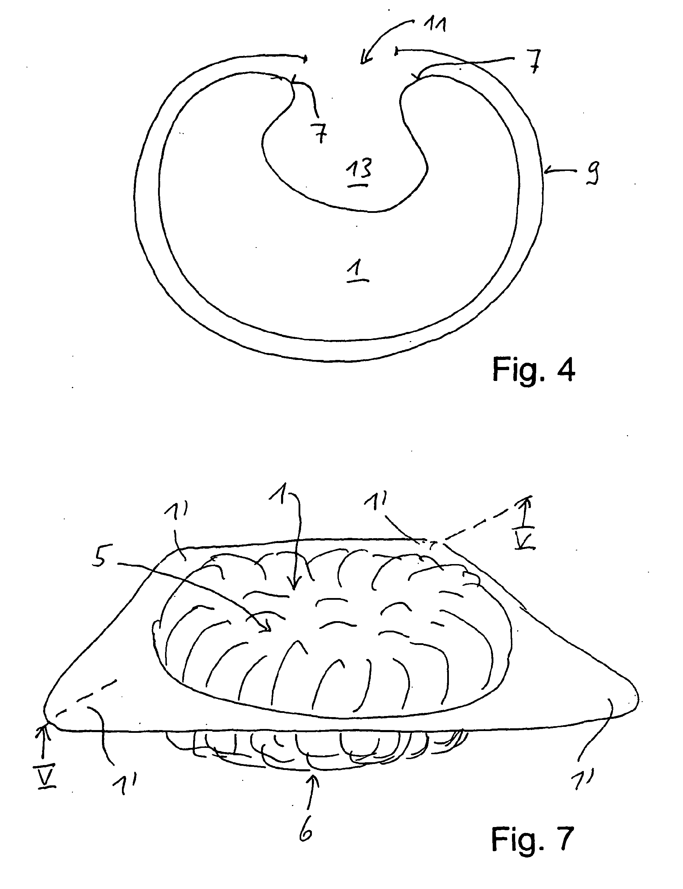

Anhand von Figur 4 ist im schematischen Querschnitt gezeigt, wie eine entsprechende Packung 1 beispielsweise nach Figur 1 oder 2 in die Außenumhüllung 9 eingelegt werden kann. Dadurch wird die Packung 1 so zusammengelegt, dass die Randbereiche 7 der Packungen 1 auf ihrer Oberseite 5 quasi zusammengeschlagen werden und hier einen Aufnahmeraum 13 bilden, auf den nachfolgend noch eingegangen wird.

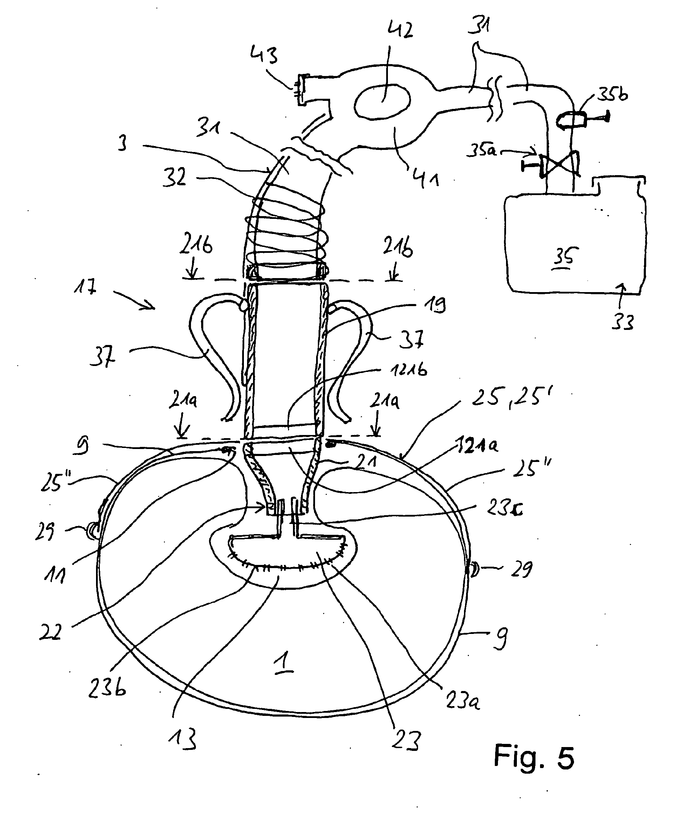

Anhand von Figur 5 ist in schematischer Seitendarstellung eine Vorrichtung zum Anlegen sowie zum Verabreichen von Packungen gezeigt.

Die Vorrichtung umfasst eine Trag- und/oder Halteeinrichtung 17, an deren Vorderseite eine entsprechende Packung 1 befestigt werden kann. Die Trag- und Halteeinrichtung 17 weist dabei einen Haltegriff oder Halteabschnitt 19 auf, an welchem die Trag- und Halteeinrichtung 17 sicher ergriffen werden kann. Diese ist bevorzugt aus Kunststoff gebildet oder umfasst Kunststoff oder eine Kunststoffumhüllung oder -isolierung, um vor allem Überhitzungen und Verbrennungen zu vermeiden.

An der Vorderseite des Haltegriffes bzw. des Halteabschnittes 19 ist dann ein eigentlicher Befestigungskopf 21 vorgesehen, der beispielsweise einen Anschluss 22 umfasst, an welchem ein Temperaturverteiler 23 bevorzugt einfach lösbar angebracht werden kann. Im gezeigten Ausführungsbeispiel ist der Temperaturverteiler 23 halbkugelförmig oder pilzförmig gestaltet. Er kann aber auch quasi glühlampenähnlich oder dergleichen gestaltet sein. Unterschiedlichste Querschnittsformen sind hier denkbar. Bevorzugt werden Querschnittsformen mit einer nach vorne hin eher konvex gestalteten Begrenzungsfläche 23b verwendet, die an der rückwärtigen Seite zu einem demgegenüber eine geringere Querschnittsabmessung aufweisenden Hals 23c übergeht. Der erwähnte Anschluss 22 kann beispielsweise aus einem Dreh- oder Bajonettverschluss bestehen, um so die Trag- und Halteeinrichtung 17 bzw. Den Befestigungskopf 21 leicht und schnell mit dem Temperaturverteiler 23 zu verbinden.

Wie aus der Querschnittsdarstellung zu ersehen ist, wird die erwähnte Trag- und Halteeinrichtung 17 mit ihrem vorne überstehenden Temperaturverteiler 23 über die erwähnte Einführöffnung 11 in die Außenumhüllung 9 eingeführt, bis der vorzugsweise pilz- oder birnenförmige Temperaturverteiler 23 in dem Aufnahmeraum 13 zu liegen kommt, und somit unmittelbar benachbart von der um den Temperaturverteiler herum gelegten Packung 1 umgeben wird.

Eine schnelle und einfache Befestigung der Trag- und Halteeinrichtung 17 an der so gebildeten mit einer Außenumhüllung 9 versehenen Packung 1 kann beispielsweise mit einer geeigneten Befestigungs- und/oder Spanneinrichtung 25 versehen sein, beispielsweise in Form einer Greifer-Krallen-Spanneinrichtung 25'. Diese Spanneinrichtung kann von der Trag- und Halteeinrichtung 17, d.h. insbesondere von dem Haltegriff 19 oder dem Befestigungskopf 21 quer weg verlaufende Befestigungs- und/oder Spannbändern 25" mit außen liegenden an der Außenumhüllung 3 verankerbaren Krallen 29 oder dergleichen bestehen. Auch eine eher flächige, beispielsweise in Draufsicht eher kreisförmige und mit der Trag- und Halteeinrichtung 17 verbundene Befestigungs- oder Spannlasche 25' kann verwendet werden, an deren Außenumfang ebenfalls wieder in Umfangsrichtung versetzt liegend mehrere Krallen 29 vorgesehen sein können, die an der Außenumhüllung 9 verankerbar sind. Möglich ist grundsätzlich auch, beispielsweise die Ecken einer Außenhülle 9 oder die Ecken einer verwendeten Packung zusammenzufassen. Auch dadurch ergibt sich durch einen auseinanderfaltbaren Griff eine gute Spannmöglichkeit.

Wie aus Figur 5 zu ersehen ist, ist die mit den Spannbändern und/oder den Krallen 29 versehene Spann- und Halteinrichtung bevorzugt am Befestigungskopf 21 angebracht und mit diesem verbunden, der an einer Schnittstelle 21a von dem Griff entfernbar ist. Dies bietet den Vorteil, dass eine Packung mit der Außenumhüllung 9 und dem Befestigungskopf 21 bevorzugt mit dem Temperaturverteiler 23 schon entsprechend vorbereitet sein kann, die im Bedarfsfall dann nur noch an ihrer Schnittstelle 21a an dem Haltegriff beispielsweise mittels eines Bajonettverschlusses-befestigt werden muss. Dies erleichtert beachtlich die Handhabung insbesondere während der Anwendung.

Der Befestigungskopf 21 bzw. der Temperaturverteiler 23 soll an der Schnittstelle 21a mit der Befestigungseinrichtung 17 bzw. dem Haltegriff oder dem Halteabschnitt 19 vorzugsweise über einen Dreh-, einen Schraub-, einen Schnapp- und/oder Rastverschluss und/oder vorzugsweise einem Bajonettverschluss verbindbar sein, also derart, dass eine einfache und schnelle Verbindung oder Trennung an dieser Stelle durch Verbinden oder Lösen zweier Befestigungsabschnitte 121a und 121b möglich ist. Dadurch können entsprechend vorbereitete Packungen mit dem Temperaturverteiler und dem Befestigungskopf vorbereitet und bei Bedarf auch während der Anwendung schnell am Haltegriff bzw. an der Halteeinrichtung ausgewechselt und angeschlossen werden.

Anstelle der erwähnten Schnittstelle 21a zwischen dem Haltegriff 19 und dem Befestigungskopf 21 bzw. dem Temperaturverteiler 23 kann eine Schnittstelle 21b auch an der zum Befestigungskopf 21 gegenüberliegenden Seite am Haltegriff oder Halteabschnitt 19 im Form einer Schnittstelle 21b ausgebildet sein. Auch dort kann ein entsprechender Dreh-, Schraub, Schnapp- und/oder Rastverschluss oder bevorzugt ein Bajonettverschluss vorgesehen sein. Auch in diesem Falle können je nach Bedarf weitere vorbereitete Packungen bereitgestellt und durch verbrauchte Packungen ausgewechselt werden. Die Schnittstelle 21b hätte den Vorteil, dass die entsprechend vorbereiteten und aufgeheizten Packungen bereits mit einem Griff 19 ergriffen und bereitgestellt werden können. Nach dem Trennen an der Schnittstelle 21b kann dann eine verbrauchte Packung mit dem Griff entfernt und eine neue Packung mit dem neuen Griff angeschraubt werden, ohne dass ein Masseur beispielsweise die überhitzte Packung selbst zum Befestigen ergreifen muss, da er die Packung über den Handgriff 19 halten kann. Auch nach Lösen an der Schnittstelle 21b kann die Packung noch über den Griff 19 gehalten und weiter eingesetzt werden, wohingegen eine zweite unterstützungsdienstleistende Person bereits eine neue vorbereitete Packung an dem Verbindungsschlauch 31 anschließt. Schließlich können aber auch beide erwähnten Schnittstellen 21a und 21b vorgesehen sein, um je nach Bedarf vor allem die untere oder obere Schnittstelle zu verwenden, um eine Packung mit oder ohne Handgriff anzuschließen. Schließlich könnte eine Schnittstelle im mittleren Bereich des Handgriffes vorgesehen sein, so dass beispielsweise der obere Teil des Handgriffes mit allen Anschlüssen der Betätigungseinrichtung von Hause aus verbunden ist und noch ein packungsseitiger Teil des Handgriffes verbleibt, der beim Abtrennen ebenfalls dazu dienen kann, die Packung noch ausreichend gut zu ergreifen.

Der Haltegriff ist dabei ferner beispielsweise so ausgeführt, dass er mit einem leichten Griff, beispielsweise einem Daumengriff oder einer Drehung so verstellt werden kann, dass die Spannung der äußeren Umhüllung 3 und damit die Packung insgesamt nachgespannt werden kann. Dies ist insbesondere deshalb von Vorteil, da während der Behandlung das Volumen der Packung 1 abnehmen kann.

Wie aus Figur 5 zu ersehen ist, geht insbesondere der Haltegriff 19 der Trag- und Halteeinrichtung 17 an der zur Packung 1 gegenüberliegenden Seite in einen Schlauch bzw. in eine schlauchartige Verbindung 31 über. Schließlich ist am Übergang von dem Haltegriff 19 zum Ansatz des davon weggehenden Schlauches 31 auch noch eine Spiralfeder 32 über eine gewisse Axiallänge vorgesehen, die als Knickschutz dient. Über den Schlauch oder die schlauchartige Verbindung 31 wird eine Verbindung beispielsweise zu einer Temperatursteuerungseinrichtung 33 geschaffen. Diese umfasst bevorzugt zumindest eine Aufheizeinrichtung zur Erzeugung von Dampf, weshalb entweder ein Wasseranschluss oder ein Wasser-Wärmebehälter 35 vorgesehen ist. Schließlich können in dieser Wärme- und/oder Kühlmittel-Aufbereitungsanlage 35 neben der zugehörigen Temperatursteuerungseinrichtung 33 noch entsprechende, der Steuerung dienende Schaltungseinheiten und Elemente vorgesehen sein, beispielsweise ein Magnetventil 35a zum Öffnen und Schließen der Verbindung der Temperatursteuerungseinheit 35 zu dem angeschlossenen Schlauch 31 und/oder eine Durchflussmengen-Einstelleinrichtung 35b und/oder weitere Ventile etc.

Schließlich sind an geeigneter Stelle der gesamten Vorrichtung, insbesondere im Bereich der Trag- und Halteeinrichtung 17 und dort wiederum insbesondere am Haltegriff bzw. am Halteabschnitt 19 ein oder mehrere Betätigungseinrichtungen bzw. Druckschalter, Druckhebel 37 etc. vorgesehen.

In einer einfachen Ausführungsform kann durch diese Betätigungseinrichtung und Hebel 37 eine Warm- oder Heißluftzufuhr oder Dampfzufuhr durch Öffnen und Schließen einer Ventil- oder Verschlusseinrichtung gesteuert werden, worüber beispielsweise in der Temperatursteuerung 33 entsprechend der voreingestellten Temperatur hierüber erzeugter Dampf (der gegebenenfalls mit weiteren Wirkstoffen versetzt sein kann) über den Schlauch 31 und die Trag- und Halteeinrichtung 17 unmittelbar dem Temperaturverteiler 23 zugeführt wird, der mit einer Vielzahl von Austrittsöffnungen und Düsen 23a versehen ist, worüber der Dampf austreten kann. Der austretende Dampf dringt dann unmittelbar durch die Oberseite 5 der am Temperaturverteiler 23 anliegenden Packung 1 ein, durchströmt die Packung 1, um dann an der Unterseite 6 der Packung durch die dort vorgesehene durchlässige Oberflächenschicht und nachfolgend durch die angrenzende ebenfalls durchlässige Außenumhüllung 9 auszutreten.

Ein Verantwortlicher kann nunmehr über die Trag- und Halteeinrichtung 17 die entsprechende Packung 1 bei Durchführung einer entsprechenden Anwendung wunschgemäß einsetzen. Dabei kann beispielsweise ein Masseur den Rücken einer Person mit der linken Hand abtasten, dabei gewisse Zonen, an denen beispielsweise Muskelverspannungen oder Verhärtungen bestehen, erkennen, um anschließend die erwähnte Packung genau über diese Zonen hinweg zu bewegen, zu drücken, gegebenenfalls für kurze Zeit zu verweilen, um hierdurch die entsprechende Anwendung der Packung verbunden mit Massageeffekten durchzuführen. Durch die erläuterte Vorrichtung ist es dabei nunmehr leicht möglich, die entsprechende Packung 1 schnell und einfach auszutauschen und beispielsweise je nach Anforderung und Wunsch zwischen einer Hochtemperaturanwendung mit dosierbarem Wärmenachschub während einem raschen Standortwechsel (gemeint sind Massagebewegungen) und Anwendungen mit niedrigerer Kontakttemperatur zu wechseln. Ein Auflegen der Packung an bestimmten Körperstellen kann durchgeführt werden. Dabei kann die Packung je nach Bedarf für bestimmte Zeitphasen an bestimmten Körperstellen belassen werden, dabei kann die Packung einmal stärker oder weniger stark angedrückt werden etc. Umgekehrt kann aber auch durch einen raschen Standortwechsel der heißen Packung, d.h. durch ein vergleichsweise schnelles über den Körper Hinwegbewegen, großflächig die Thermorezeptoren angesprochen werden. Der Körper reagiert dabei mit erhöhtem Wärmeabtransport über das Herz-Kreislauf-System. Legt man die Packung beispielsweise an einer bestimmten Stelle des Körpers ab, nachdem man sie vorher über verschiedene Körperstellen hinweggeführt hat, meint der Körper nunmehr, dass ihm ein Wärmeangebot auch in jenem Bereich zugeführt wird, über welchen vorher die Packung hinweggeführt wurde. Tatsächlich wird aber in diesem Bereich vom Körper Wärme abgeführt. Um unter diesen Bedingungen eine Verkühlung zu vermeiden, kann ferner eine großflächige Abdeckung mit einer leicht gewichtigen Reflexionsfolie verwendet werden, die beispielsweise eine Alubeschichtung aufweist. Diese Alufolie wird während einer Massagebewegung sektorweise immer wieder zu jenen Bereichen verschoben, an denen die Packung gerade nicht eingesetzt wird. Nach Beendigung der Massage und dem Ablegen der Packung kann die betreffende Folie ganz über den Körper eines Gastes gelegt werden, bezüglich dem die Packungsanwendung durchführt wird.

Mittels der erwähnten Betätigungseinrichtung oder -schalter oder -hebel 37 ist es aber beispielsweise auch möglich, durch eine geeignete Auslöseeinrichtung direkt Schalterveränderungen an der Temperatursteuerungseinrichtung oder dem Temperatursteuerungsgerät 33 vorzunehmen. Dies kann beispielsweise durch einen nicht-elektrischen Auslöser, durch-Sende- oder Funksignale oder beispielsweise über eine Niederspannungsleitung 34 ausgelöst und/oder übermittelt werden, die bevorzugt vom Haltegriff 19 zur Temperatursteuerungseinrichtung 33 verläuft. Im Falle einer Schnittstelle 21b umfasst diese auch eine Trennungs- bzw. Koppelmöglichkeit für die Niederspannungsleitung 34. Ergänzend können beispielsweise auch Potentiometer oder weitere Durchflussregler vorgesehen sein, um nicht nur die Temperatursteuerung entsprechend vorzunehmen, sondern auch um die zugeführte Menge der erwärmten oder gekühlten Luft, des Dampfes oder eines fließfähigen Mediums in gewünschten Grenzen zu dosieren.

Dabei kann beispielsweise zielgerichtet umgeschaltet werden, um mehr oder weniger warme oder heiße Luft oder Dampf zuzuführen. Möglicherweise kann auch dadurch umgeschaltet werden, dass zumindest kurzweilig kühle Luft über den Temperaturverteiler 23 und damit über die Packung 1 abgeben wird, um Temperaturwechsel-Anwendungen durchzuführen. Gegebenenfalls könnte zumindest kurzweilig auch oder bei Bedarf Wasser oder Heißwasser oder sonstiges fließfähiges und nicht nur gasfähiges Medium von dem Gerät 33 über den Schlauch 31 angefordert werden, die dann über den Temperaturverteiler 23 und die Packung 1 abgegeben wird.

Bei Bedarf könnten anstelle eines einzigen Schlauches auch mehrere Schläuche vorgesehen sein, an denen getrennt beispielsweise heiße Luft, Dampf und Heißwasser oder sonstige Lösungen zuführbar sind, so dass durch Betätigung der Hebel 37 unmittelbar die gewünschten Medien gegebenenfalls über getrennte Schläuche oder Kammern in einem Schlauchsystem über den nachfolgenden Temperaturverteiler 23 an die Packung und damit an einen Gast abgegeben werden können, der eine entsprechende Anwendung an sich durchführen lässt.

Bevorzugt ist der Haltegriff und Verbindungsschlauch drucklos gehalten, um eine schnelle An- und Abkopplung vor allem an dem Temperaturverteilerkopf 23 zu gewährleisten. Alternativ wäre auch möglich, dass vor dem Temperaturverteilerkopf 23 ein Ventil in der Trag- und Halteeinrichtung 17 integriert ist, welches automatisch verschlossen wird, wenn der Temperaturverteilerkopf 23 an oder abgekoppelt wird. Bei Bedarf können auch mehrere Temperaturverteilerköpfe 33 verwendet werden, die bereits mit den Packungen 1 in der geschilderten Weise verbunden sind. Dabei können die Packungen schon vorbefeuchtet und/oder mit Öl versetzt worden sein, so dass während der Anwendung schnell eine aufgebrauchte Packung 1 durch eine andere Packung mit einem bereits integriert vorbereiteten Befestigungskopf 21, also mit dem Temperaturverteiler 23 an der Trag- und Halteeinrichtung 17 angeschlossen werden kann.

Bei Bedarf kann auch an geeigneter Stelle im Verlauf des Schlauchabschnittes und/oder im Bereich der eigentlichen Halte- und Trageinrichtung 17 eine Wirkstoffkammer 41 zwischengeschaltet sein, in welche beispielsweise eine Wirkstoff- oder Kräuterfüllung 42 etc. einsetzbar ist. Die Wirkstoffkammer 41 soll dabei so beschaffen sein, dass das über den Schlauch zugeführte Medium, beispielsweise Dampf, diese Wirkstoffkammer 41 durchströmt und beispielsweise über einen separaten Auslass 43 gasförmige Wirkstoffe mit abgegeben werden, um die Luft entsprechend mit Wirkstoffen zu durchsetzen. Genauso kann das hierüber zugeführte Medium, beispielsweise heiße Luft, heißer Dampf oder gegebenenfalls auch heißes fließfähiges Medium wie Wasser durch diese Wirkstoffkammer hindurch strömen und mit den Wirkstoffen versetzt dann weiter der Packung 1 zugeführt werden. Alternativ oder ergänzend kann insbesondere bei Verwendung von heißem Dampf oder heißer Luft diese Wirkstoffkammer über den erwähnten separaten Auslass 43 verfügen, um den entsprechenden Dampf oder die heiße Luft vor allem mit Wirkstoff versetzt in ihre Umgebung abzugeben.

Der Vollständigkeit halber wird auch erwähnt, dass beispielsweise auch ergänzend eine elektrische Heizeinrichtung vorgesehen sein kann, insbesondere im Bereich des Haltegriffes und/oder der Befestigungseinrichtung. Hierüber zuströmendes Medium kann darüber noch ergänzend aufgeheizt werden. Möglich wäre sogar, dass der Befestigungskopf 21 mit einer integrierten elektrischen Heizung versehen ist, um hierüber beispielsweise die Packung 1 aufzuheizen. Bevorzugt an der Schnittstelle 21a wäre dann eine elektrische Kontaktierung vorgesehen, um bei Anschluss des Befestigungskopfes 21 an die Halteeinrichtung 19 den elektrischen Kontakt zur Energieversorgung für die integrierte Aufheizeinrichtung herzustellen.

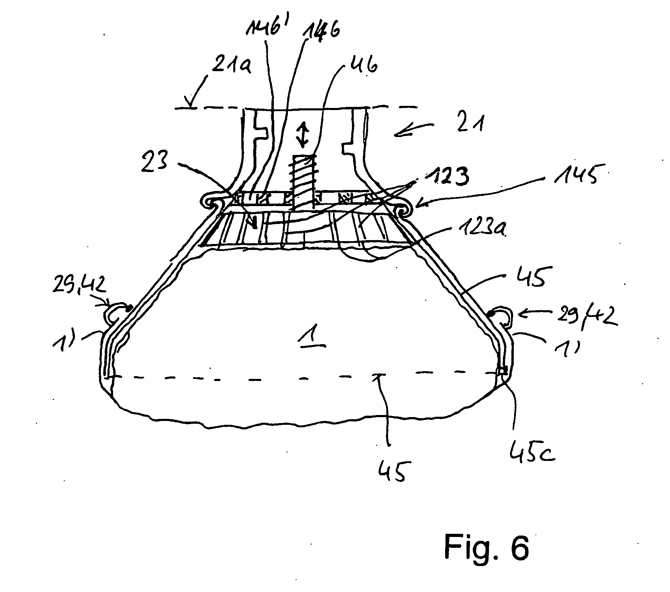

Nachfolgend wird auf ein erfindungsgemäßes Ausführungsbeispiel gemäß Figur 6 und 7 eingegangen. In Figur 6 ist in schematischer axialer Querschnittsdarstellung ein abgewandelter Befestigungskopf 21 mit einem Temperaturverteiler 23 gezeigt.

Der Befestigungskopf 21 nach Figur 6 weist eine trichterförmige Aufnahme 45 auf, die gegenüberliegend zu ihrer in Figur 6 gezeigten unten liegenden Öffnung am rückwärtigen Bereich über die erwähnte Schnittstelle 21a verfügt, beispielsweise in Form eines Bajonettverschlusses (oder in Form eines anderen geeigneten Verbindungsanschlusses, wie anhand der vorausgegangenen Ausführungsbeispiele bereits erläutert wurde).

Der Befestigungskopf 21 umfasst neben der trichterförmigen Aufnahme 45 und dem Befestigungsanschluss 121a den erwähnten Temperaturverteiler 23, der ebenfalls fest integriert in der trichterförmigen Aufnahme 45 eingebaut ist. Dieser Temperaturverteiler 23 weist ebenfalls eine Vielzahl von an der Austrittsseite vorgesehenen Öffnungen oder Düsen 23a oder sonstige geeignete Perforation auf, damit das entsprechende Wärmemedium hier austreten kann. Der Temperaturverteiler kann dabei mit integrierten Verteilerdüsen 123a versehen sein, wie aus der Schnittdarstellung gemäß Figur 6 ersichtlich. Das verteilsieb in Form des Temperaturverteilers 23 kann dabei an der Innenseite des Trichters entsprechend befestigt sein. Möglich ist aber auch eine Konstruktion entsprechend Figur 6, bei welcher der Temperaturverteiler 23 über einen rückwärtigen Gewindestab 46 in eine auch als Aufnahme oder Brücke zu bezeichnende Halterung 146 verdrehbar ist und damit in Axialrichtung weiter nach unten ausgedreht werden kann. Die Anordnung ist dabei derart, dass die trichterförmige Aufnahme 45 beispielsweise am rückwärtigen Bereich mit einer horizontal umlaufenden Bördelung 145 oder einer vergleichbaren Einrichtung versehen ist, so dass der untere Teil der trichterförmigen Aufnahme 45 gegen den oberen, mit dem Bajonettverschluss 21 versehenen Teil um die zentrale Längsachse verdreht werden kann. Mit dieser Verdrehbewegung wird dann der Temperaturverteiler 23, der mit der trichterförmigen Aufnahme 45 drehfest verbunden ist, mit verdreht, so dass bei feststehender rückwärtiger Halterung oder Brücke 146 nunmehr der Gewindestab 46 weiter ein- oder ausgedreht wird, damit der Temperaturverteiler nach unten wandert. Durch eine derartige Verdrehbewegung kann dadurch nunmehr ein zunehmender Druck auf die Packung 1 ausgeübt werden, so dass die Packung entsprechend fest eingespannt und zu einem gewissen Maße verdichtet wird. Der Gegendruck wird dadurch durch die Außenumhüllung oder durch die die Packung aufnehmende Halterung aufgenommen, die durch die nachfolgend noch erwähnten Tuch- oder Eckabschnitte 1' am Trichter gehalten wird. Hier könnte auch eine innenliegende Verstellbewegung vorgesehen sein, dass beispielsweise über einen Einstellgriff oder -hebel von der Griffeinrichtung ausgehend, beispielsweise über den erwähnten Temperaturverteiler, ein unterschiedlich einstellbarer Druck auf die Packung wirkt.

Auch die erwähnte Halterung oder Brücke 146 ist natürlich mit entsprechenden Durchbrüchen oder Kanälen 146' versehen, damit das entsprechende, vor allem aufgeheizte Medium zum nachfolgenden Temperaturverteiler und durch die dort vorgesehenen Kanäle 123 an den unten liegenden Austrittsöffnungen oder Düsen 123a austreten und in die Packung eintraten kann.

Durch diese Anordnung wird innerhalb der trichterförmigen Aufnahme 45 (die natürlich auch mit anderen Formgebungen gestaltet sein kann) ein nach zum rückwärtigen Anschlussende hin durch den Temperaturverteiler 23 begrenzter Aufnahmeraum geschaffen, in welchem eine Packung 1 eingesetzt werden kann.

Eine geeignete Packung ist beispielsweise in schematischer perspektivischer Darstellung anhand von Figur 7 wiedergegeben. Die Packung weist dabei näherungsweise eine eher ballige Form auf, wobei in Draufsicht die Randbegrenzung beispielsweise nach Art eines viereckigen oder quadratischen Tuches gebildet sein kann. Das eigentliche Packungsmaterial ist in Draufsicht eher in einem mittleren runden oder kreisförmigen Bereich untergebracht, so dass sich ein eher flacher Packungsrand mit in den Eckbereichen stärker überstehenden Tuch- oder Eckabschnitten 1' ergibt.

Eine so gebildete Packung kann dann mit ihrem balligen Packungsabschnitt von unten her über die Öffnung in den trichterförmigen Aufnahmeraum eingefügt werden, bis die Packung beispielsweise mit ihrer Oberseite 6 unmittelbar an der Düsenaustrittsöffnung des Temperaturverteilers 23 anliegt. Die überstehenden Laschenbereiche 1' werden dann außenliegend auf die Außenseite der trichterförmigen Aufnahme 45 angelegt, wie dies in der schematischen Querschnittsdarstellung gemäß Figur 6 ersichtlich ist. Dabei zeigt Figur 6 eine Querschnittsdarstellung durch die eingelegte Packung längs der strichlierten Linie V-V in Figur 7. Die außenliegenden Laschenbereiche 1' der Packung können dann durch die erwähnten Befestigungsmechanismen beispielsweise mittels der an der Außenseite der trichterförmigen Aufnahme ausgebildeten oder verankerten Laschen befestigt werden, die am unten liegenden freien Ende mit Krallen oder klettartigen Verschlüssen etc. versehen sind, die dann mit den entsprechenden Laschen 1' zusammenwirken und diese halten. Genauso können am Außenumfang der trichterförmigen Aufnahme entsprechende Klemmeinrichtungen 47 ausgebildet sein, in welche die entsprechenden Eckbereiche oder Laschen 1' der Packung eingeklemmt werden. Die Ecklaschen 1' werden insoweit auch als Dichtungslaschen bezeichnet. Sie können dazu auch mit wulstförmigen Erhebungen versehen sein, die die Klemmwirkung verbessern. Dies ist in Figur 6 nur rein schematisch angedeutet.

Wird eine Packung derart in die trichterförmige Aufnahme eingesetzt, so ragt nach unten hin über den unteren Rand 45c die Packung in entsprechend ausreichendem Ausmaß über, so dass eine entsprechend hierüber gehaltene Packung zum Anschluss an die Halteeinrichtung problemlos über den Körper eines Gastes zur Durchführung einer Anwendung hinweg geführt werden kann, ohne dass der Trichterrand 45c den Körper eines Gastes oder Patienten berührt.

Aber auch bei diesem Ausführungsbeispiel kann die erwähnte trichterförmige Aufnahme 45 bereits von Hause aus mit einem Haltegriff 19 versehen sein, insbesondere dann, wenn eine lösbare Schnittstelle 21b oberhalb des Handgriffes, also auf der zur trichterförmigen Aufnahme gegenüberliegenden Seite des Griffes oder im mittleren Bereich des Griffes vorgesehen ist. Dadurch können die entsprechend vorbereiteten Packungen gut ergriffen und an der Schnittstelle 21b mit einem entsprechenden Anschlussmechanismus, vorzugsweise am Ende eines Verbindungsschlauches, angeschlossen werden.

Nachfolgend wird auf einen typischen Behandlungsverlauf eingegangen, wie er insbesondere mit der erfindungsgemäßen Vorrichtung möglich ist.

Üblicherweise wird eine Packung beispielsweise für 10 Minuten in ein warmes Wasserbad gelegt, um die Packung einzuweichen. Das Wasser weist dabei üblicherweise eine Wassertemperatur von ca. 40°C auf. Anschließend wird die Packung oberhalb eines Wasserbades ebenfalls für etwa 10 Minuten bedampft, wobei die Temperatur bis auf eine maximale Behandlungstemperatur von etwa 65°C (also allgemein zwischen 60°C und 70°C) erhöht wird. Jedesmal, wenn der Bereich von etwa 55°C bis 65°C für die Packung überschritten bzw. unterschritten wird, kommt die sogenannte Schmelzwärme zum Tragen (dies betrifft den Übergang von der wässrigen in die dampfförmige Phase, bei welcher in den Packungen befindliche Wirkstoffe wie beispielsweise Cumarin in die gasförmige Phase übergehen). Man spricht hier auch von dem Temperaturbereich, in welchem die ätherischen Öle in Lösung gehen. Im nachfolgenden Zeitraum von beispielsweise 20 bis 30 Minuten findet dann der erste Behandlungsabschnitt statt, wobei unter Umständen die Temperatur in der Packung langsam zu sinken beginnt. Durch die vorhergehende Bedampfung in der wässrigen Phase sind, wie erwähnt, die ätherischen Öle in die dampfförmige Phase übergegangen. Sinkt die Temperatur in der Packung unter 65°C, so wird die Dampfphase wieder unterschritten und die Wirkstoffe liegen wiederum in wässriger Lösung vor.

Beispielsweise in dem nachfolgenden Zeitraum von 30 bis 40 Minuten kann dann vor allem im Gegensatz zum Stand der Technik aufgrund der beschriebenen Vorrichtung nunmehr eine erneute Aufwärmung der Packung durchgeführt werden. Am Haltegriff kann die Dampfzufuhr durch die dort vorgesehenen Betätigungselemente eingeschaltet werden. Die Temperatur steigt wiederum an und die ätherischen Öle wechseln von ihrer wässrigen in ihre dampfförmige Phase. Diese Wechsel können bei Bedarf häufiger vorgenommen werden. Ebenso kann durch die häufige Zuschaltung der Aufwärmeinrichtung ein eher gleichmäßiger Temperaturverlauf beibehalten werden. Beliebige Variationsmöglichkeiten sind hier gegeben.

Die anhand von Figur 7 gezeigten und erwähnten Eckbereiche oder -laschen 1', die Teil der Packung selbst oder Teil der Packungsumhüllung sind, können aber lediglich Teil einer eher tuch- oder folienförmigen, beispielsweise eher quadratischen Außenumhüllung 3 sein. In diesem Fall muss eine entsprechende Packung 1 in dem Ausführungsbeispiel gemäß Figur 6 lediglich in die trichterförmige Aufnahme 45 eingelegt werden, um dann diese Packung durch eine von unten her übergestülpte Außenumhüllung 3 mittels den umgelegten Eckbereichen 1' unter Verwendung der erwähnten Klemmeinrichtung 47 an der Außenseite der trichterförmigen Aufnahme 45 zu befestigen. Dadurch kann eine entsprechende Packung gehalten und gesichert werden, wobei die Außenumhüllung, wie bereits erläutert, entsprechend perforiert und/oder durchlässig gestaltet ist, zumindest für dampf- und/oder wässrige Lösungen.

Alternativ zur vorstehend erläuterten Befestigung der Packung 1 kann aber auch auf die erwähnte Außenumhüllung 3 verzichtet werden, insbesondere dann, wenn an der Packung von Hause aus zumindest in einzelnen umlaufenden Abschnitten sogenannte Einspann- oder Eckbereiche 1' vorgesehen sind, mittels der die Packung direkt (also ohne Verwendung einer Außenumhüllung) entsprechend verankert werden kann, insbesondere an der erwähnten trichterförmigen Aufnahme 45.

Insbesondere dann, wenn eine Außenumhüllung verwendet wird, kann diese gegebenfalls aus unterschiedlichen Materialien bestehen und/oder mit unterschiedlichen Oberflächen versehen sein, beispielsweise glatte oder raue Oberflächen aufweisen, um hier im Einsatz unterschiedliche Reibmomente auf die Haut auszuüben, also die Mechanik bei der Behandlung zu verhindern. Insoweit soll beispielsweise auf ein Peel-Effekt verwiesen werden.

## Patentansprüche

1. Vorrichtung zum Anlegen und/oder Verabreichen von Packungen,
- mit einer Trag- und Halteeinrichtung (17) zur Befestigung einer Packung (1),
- wobei die Trag- und Halteeinrichtung (17) dazu einen Befestigungskopf (21), an welchem die Packung (1) befestigbar ist, umfasst,
- wobei der Befestigungskopf (21) einen Temperaturverteiler (23), der von dem Dampf in Richtung Packung (1) durchströmbar ist, umfasst,
- wobei der Befestigungskopf (21) ferner eine Aufnahme (45) mit einem Aufnahmeraum und einer Öffnung, worüber die Packung (1) in den Aufnahmeraum einführbar ist, umfasst,
- wobei der Aufnahmeraum zum rückwärtigen Anschlussende hin durch den Temperaturverteiler (23) begrenzt ist,
- mit einer Temperatursteuerungseinrichtung (33), von der aus flüssiges und/oder gasförmiges Medium und/oder Dampf durch einen Schlauch (31) oder eine schlauchartige Verbindung (31) und den Temperaturverteiler (23) einer in dem Befestigungskopf (21) befestigten Packung (1)zuführbar ist,
- wobei die Packung mittels der Temperatursteuerungseinrichtung durch das der Packung (1) zuführbare flüssige und/oder gasförmige Medium und/oder den der Packung (1) zuführbaren Dampf erwärm- und/oder kühlbar oder auf ein bestimmtes Temperaturniveau oder einen bestimmten Temperaturverlauf einstellbar ist, und
- wobei die Trag- und Halteeinrichtung (17) einen Haltegriff oder Halteabschnitt aufweist, worüber die befestigte Packung (1) zur Durchführung einer Anwendung lageveränderlich bewegbar und/oder positionierbar ist.

2. Vorrichtung nach Anspruch 1, **dadurch gekennzeichnet, dass** der Befestigungskopf (21) oder der Temperaturverteiler (23) anwendungsseitig eine konvexe Oberfläche aufweist.

3. Vorrichtung nach einem der Ansprüche 1 oder 2, **dadurch gekennzeichnet, dass** der Befestigungskopf (21) oder der Temperaturverteiler (23) im Querschnitt pilz- oder birnenförmig gestaltet ist.

4. Vorrichtung nach einem der Ansprüche 1 bis 3, **dadurch gekennzeichnet, dass** der Betfestigungskopf (21) bzw. der Temperaturverteiler (23) mit einer Vielzahl von Austrittsöffnungen oder -düsen (23) versehen ist.

5. Vorrichtung nach einem der Ansprüche 1 bis 4, **dadurch gekennzeichnet, dass** die Trag- und Halteeinrichtung (17) und/oder der Haltegriff oder Halteabschnitt isoliert ist oder zumindest isolierende Abschnitte umfasst.

6. Vorrichtung nach einem der Ansprüche 1 bis 5, **dadurch gekennzeichnet, dass** zumindest eine lösbare Schnittstelle (21a, 21b) vorgesehen ist, worüber der Befestigungskopf (21) und/oder der Temperaturverteiler (23) direkt oder zumindest mittelbar mit einer Zuführeinrichtung und insbesondere mit einem Schlauch (31) verbindbar ist.

7. Vorrichtung nach Anspruch 6, **dadurch gekennzeichnet, dass** die lösbare Schnittstelle (21a) zwischen dem Befestigungskopf (21) und/oder dem Temperaturverteiler (23) und der Halte- und Befestigungseinrichtung (17), insbesondere dem Haltegriff oder Halteabschnitt (19) vorgesehen ist.

8. Vorrichtung nach Anspruch 6 oder 7, **dadurch gekennzeichnet, dass** die lösbare Schnittstelle (21b) an der zum Befestigungskopf (21) und/oder zum Temperaturverteiler (23) gegenüberliegenden Seite der Halte- und Befestigungseinrichtung (17) oder in einem mittleren Bereich der Halte- und Befestigungseinrichtung (17) dazu vorgesehen ist.

9. Vorrichtung nach einem der Ansprüche 6 bis 8, **dadurch gekennzeichnet, dass** die Schnittstelle (21a, 21b) aus einem Schraub-, einem Schnapp-, einem Rast- und/oder einem Bajonettverschluss besteht.

10. Vorrichtung nach einem der Ansprüche 1 bis 9, **dadurch gekennzeichnet, dass** an der Trag- und Halteeinrichtung (17), vorzugsweise an dem an der Trag- und Halteeinrichtung (17) ausgebildeten Haltegriff oder Halteabschnitt(19) zumindest ein oder mehrere Betätigungseinrichtungen oder -hebel (37) zur Steuerung der Medienzufuhr oder Dampfzufuhr und/oder der Steuerung der Temperatur und/oder der Menge des zugeführten Medium oder Dampfes vorgesehen ist.

11. Vorrichtung nach einem der Ansprüche 1 bis 10, **dadurch gekennzeichnet, dass** die Trag- und Halteeinrichtung (17) mit Betätigungseinrichtung und/oder -hebeln (37) versehen ist, worüber die von der Trag- und Halteeinrichtung (17) entfernt positionierte und über zumindest eine Schlauchverbindung (31) verbundene Temperatursteuerungseinrichtung (33) zur entsprechend gesteuerten Aufheizung und/oder Kühlung und/oder zur Steuerung der Zufuhr und/oder Dosierung der zugeführten Menge eines Aufheiz- oder Kühlmediums steuerbar ist.

12. Vorrichtung nach Anspruch 11, **dadurch gekennzeichnet, dass** die Temperatursteuerungseinrichtung (33) über ein nicht elektrisches Kabel, über eine elektrische Verbindung, vorzugsweise eine Niedervolt-Verbindung und/oder über Funk steuerbar ist.

13. Vorrichtung nach einem der Ansprüche 1 bis 12, **dadurch gekennzeichnet, dass** der Befestigungskopf (21) eine trichterförmige Aufnahme (45) umfasst.

14. Vorrichtung nach Anspruch 13, **dadurch gekennzeichnet, dass** auf der Außenseite der trichterförmigen Aufnahme (45) Befestigungs- und/oder Klemmeinrichtungen (47) vorgesehen sind, worüber an der einzufügenden Packung (1) ausgebildete Laschen oder Eckbereiche (1') und damit eine eingelegte Packung befestigbar ist.

15. Vorrichtung nach einem der Ansprüche 1 bis 14, **dadurch gekennzeichnet, dass** in der schlauchartigen Verbindung (31) zwischen der Temperatursteuerungseinrichtung (33) und dem Befestigungskopf (21) zumindest eine Wirkstoffkammer (41) zwischengeschaltet ist, worüber Wirkstoffe dem durch die schlauchartige Verbindung (31) durchströmenden Medium zuführbar sind.

16. Vorrichtung nach einem der Ansprüche 1 bis 15, **dadurch gekennzeichnet, dass** die Packung (1) mittels einer Befestigungs- und/oder Spanneinrichtung (25) mit der Trag- und Halteeinrichtung (17) verbindbar ist.

17. Vorrichtung nach Anspruch 16, **dadurch gekennzeichnet, dass** die Packung (1) eine Außenumhüllung (3) umfasst oder in eine beutelförmigen Außenumhüllung (9) gebbar ist, in welcher der Befestigungskopf (21) und/oder der Temperaturverteiler (23) positionierbar ist.

18. Vorrichtung nach Anspruch 16 oder 17, **dadurch gekennzeichnet, dass** die Befestigungs- und/oder Spanneinrichtung (25) Befestigungs- oder Spannbänder (27) oder eine Befestigungs- oder Spannlasche (27') umfasst, an deren Außenumfang vorzugsweise Krallen- oder Greifabschnitte eines Klettverschlusses (29) oder dergleichen vorgesehen sind, die mit entsprechenden Verankungerungs- oder Greifabschnitten an der Außenumhüllung (3) der Packung (1) oder der die Packung (1) aufnehmenden Außenumhüllung (9) zur Befestigung der Packung (1) wechselwirken.

19. Vorrichtung nach einem der Ansprüche 1 bis 18, **dadurch gekennzeichnet, dass** die Temperatursteuerungseinrichtung (33) eine elektrische Aufheizeinrichtung umfasst, worüber die Packung (1) direkt oder mittelbar aufheizbar ist.

20. Vorrichtung nach Anspruch 13 oder einem der Ansprüche 14 bis 19 in Verbindung mit Anspruch 13, **dadurch gekennzeichnet, dass** die trichterförmige Aufnahme (45) quer zur Längserstreckung einen kreisförmigen, ovalen oder n-polygonalen Querschnitt oder einen dieser Form angenäherten Querschnitt aufweist.

21. Vorrichtung nach einem der Ansprüche 1 bis 20, **dadurch gekennzeichnet, dass** die Vorrichtung mit einer Druckbeaufschlagungseinrichtung zur Druckbeaufschlagung einer Packung (1) versehen ist, vorzugsweise in Form einer hierfür vorgesehenen Aufnahme (45), insbesondere in Trichterform.

22. Vorrichtung nach Anspruch 21, **dadurch gekennzeichnet, dass** die Druckbeaufschlagungseinrichtung einen Schraub- oder Drehmechanismus umfasst, worüber eine Druckvorrichtung, vorzugsweise in Form eines Temperaturverteilers (23), in der Aufnahme (45) auf die Packung (1) zu verstellbar ist.

23. Vorrichtung nach Anspruch 21 oder 22, **dadurch gekennzeichnet, dass** die vorzugsweise trichterförmige Aufnahme (45) zumindest zweigeteilt ist und einen drehbaren, vorzugsweise unteren Abschnitt aufweist, worüber die Druckbeaufschlagungseinrichtung in Abhängigkeit der Drehbewegung des vorzugsweise unteren Teils der Aufnahme (45) axial verstellbar ist.

## Claims

1. Device for applying and/or administering packs,
- comprising a carrying and holding means (17) for attaching a pack (1),
- wherein the carrying and holding means (17) comprises for this purpose an attachment head (21) to which the pack (1) can be attached,
- wherein the attachment head (21) comprises a temperature distributor (23) through which steam can flow towards the pack (1),
- wherein the attachment head (21) further comprises a recess (45) having a receiving chamber and an opening via which the pack (1) can be introduced into the receiving chamber,
- wherein the receiving chamber is delimited towards the rear fastening end by the temperature distributor (23),
- comprising a temperature control means (33), from which a liquid and/or gaseous medium and/or steam can be introduced, via a tube (31) or a tube-like connection (31) and the temperature distributor (21), to a pack (1) attached in the attachment head (21),
- wherein it is possible to heat and/or cool the pack to a particular temperature or in accordance with a particular temperature curve using the temperature control means, by means of the liquid and/or gaseous medium which can be introduced to the pack (1) and/or the steam which can be introduced to the pack (1),
- wherein the carrying and holding means (17) comprises a handle or holding portion, via which the attached pack (1) can be moved and/or positioned so as to change location, for application.

2. Device according to claim 1, **characterised in that** the attachment head (21) or the temperature distributor (23) has a convex surface on the application side.

3. Device according to either claim 1 or claim 2, **characterised in that** the attachment head (21) or the temperature distributor (23) has a mushroom-shaped or pear-shaped cross-section.

4. Device according to any one of claims 1 to 3, **characterised in that** the attachment head (21) or the temperature distributor (23) is provided with a plurality of outlet openings or nozzles (23).

5. Device according to any one of claims 1 to 4, **characterised in that** the carrying and holding means (17) and/or the handle or holding portion is insulated or at least comprises insulating portions.

6. Device according to any one of claims 1 to 5, **characterised in that** at least one releasable interface (21 a, 21 b) is provided, via which the attachment head (21) and/or the temperature distributor (23) can be connected directly or at least indirectly to an introduction means and in particular to a tube (31).

7. Device according to claim 6, **characterised in that** the releasable interface (21 a) is provided between the attachment head (21) and/or the temperature distributor (23) and the holding and attachment means (17), in particular the handle or holding portion (19).

8. Device according to either claim 6 or claim 7, **characterised in that** the releasable interface (21 b) is provided for on the side of the holding and attachment means (17) remote from the attachment head (21) and/or the temperature distributor (23) or in a central region of the holding and attachment means (17).

9. Device according to any one of claims 6 to 8, **characterised in that** the interface (21 a, 21 b) consists of a screw, a snap, a catch and/or a bayonet connection.

10. Device according to any one of claims 1 to 9, **characterised in that** at least one or more actuation means or levers (37), for controlling the introduction of medium or steam and/or controlling the temperature and/or the amount of medium or steam introduced, are provided on the carrying and holding means (17), preferably on the handle or holding portion (19) formed on the carrying and holding means (17).

11. Device according to any one of claims 1 to 10, **characterised in that** the carrying and holding means (17) is provided with actuation means and/or levers (37) by means of which the temperature control means (33), positioned at a distance from the carrying and holding means (17) and connected via at least one tube connection (31), can be controlled for correspondingly controlled heating and/or cooling and/or for controlling the supply and/or metering of the supplied amount of a heating or cooling medium.

12. Device according to claim 11, **characterised in that** the temperature control means (33) can be controlled via a non-electric cable, via an electrical connection, preferably a low-voltage connection, and/or wirelessly.

13. Device according to any one of claims 1 to 12, **characterised in that** the attachment head (21) comprises a funnel-shaped recess (45).

14. Device according to claim 13, **characterised in that** attachment and/or fixing means (47), by means of which straps or corner regions (1') formed on the pack (1) to be introduced, and thus an inserted pack, can be attached, are provided on the outside of the funnel-shaped recess (45).

15. Device according to any one of claims 1 to 14, **characterised in that** in the tube-like connection (31), at least one active substance chamber (41) is interposed between the temperature control means (33) and the attachment head (21), via which chamber active substances can be introduced to the medium flowing through the tube-like connection (31).

16. Device according to any one of claims 1 to 15, **characterised in that** the pack (1) can be connected to the carrying and holding means (17) by means of an attachment and/or fixing means (25).

17. Device according to claim 16, **characterised in that** the pack (1) comprises an outer sheath (3) or can be placed in a pouch-shaped outer sheath (9) in which the attachment head (21) and/or the temperature distributor (23) can be positioned.

18. Device according to either claim 16 or claim 17, **characterised in that** the attachment and/or fixing means (25) comprises attachment or fixing bands (27) or an attachment or fixing strap (27'), on the outer periphery of which claw or grip portions of a hook-and-loop fastener (29) or the like are preferably provided, which interact with corresponding anchoring or grip portions on the outer sheath (3) of the pack (1), or on the outer sheath (9) which receives the pack (1), to attach the pack (1).

19. Device according to any one of claims 1 to 18, **characterised in that** the temperature control means (33) comprises an electrical heating means which can heat the pack (1) directly or indirectly.

20. Device according to claim 13 or any one of claims 14 to 19 in connection with claim 13, **characterised in that** the funnel-shaped recess (45) comprises a circular, oval or n-polygonal cross-section or a cross-section approximating one of these shapes transverse to the longitudinal extension.

21. Device according to any one of claims 1 to 20, **characterised in that** the device is provided with a pressurisation means for pressurising a pack (1), preferably in the form of a recess (45), in particular in a funnel shape, provided for this purpose.

22. Device according to claim 21, **characterised in that** the pressurisation means comprises a screwing or turning mechanism, by means of which a pressure device, preferably in the form of a temperature distributor (23), can be adjusted to the packing (1) in the recess (45).

23. Device according to either claim 21 or claim 22, **characterised in that** the preferably funnel-shaped recess (45) is divided at least in two and comprises a rotatable, preferably lower portion by means of which the pressurisation means can be adjusted axially as a function of the rotational movement of the preferably lower part of the recess (45).

## Revendications

1. Dispositif pour appliquer et/ou administrer des enveloppements,
- comprenant un moyen de support et de maintien (17) pour la fixation d'un enveloppement (1),
- dans lequel le moyen de support et de maintien (17) comprend à cet effet une tête de fixation (21) sur laquelle l'enveloppement (1) peut être fixé,
- la tête de fixation (21) comprenant un répartiteur de température (23) qui peut être traversé par la vapeur en direction de l'enveloppement (1),
- la tête de fixation (21) comprenant en outre un récepteur (45) avec une chambre de réception et une ouverture via laquelle l'enveloppement (1) peut être introduit dans la chambre de réception,
- la chambre de réception étant limitée par le répartiteur de température (23) vers l'extrémité postérieure,
- comprenant un système de commande de température (33) depuis lequel un milieu liquide et/ou gazeux et/ou de la vapeur peut être amené(e), via un tuyau (31) ou une liaison (31) semblable à un tuyau et via le répartiteur de température (23), à un enveloppement (1) fixé dans la tête de fixation (21),
- l'enveloppement pouvant être réchauffé et/ou refroidi au moyen du système de commande de température par le milieu liquide et/ou gazeux amené à l'enveloppement (1) ou la vapeur amenée à l'enveloppement (1), ou être réglé à un niveau de température déterminée ou une évolution de température déterminée, et
- le moyen de support et de maintien (17) comprend une poignée de maintien ou un tronçon de maintien au moyen de laquelle/duquel l'enveloppement fixé (1) est déplaçable et/ou susceptible d'être positionné en modifiant sa position pour l'exécution d'une application.

2. Dispositif selon la revendication 1, **caractérisé en ce que** la tête de fixation (21) ou le répartiteur de température (23) comporte du côté application une surface convexe.

3. Dispositif selon l'une ou l'autre des revendications 1 ou 2, **caractérisé en ce que** la tête de fixation (21) ou le répartiteur de température (23) est conçu(e) avec une section transversale en forme de champignon ou en forme de poire.

4. Dispositif selon l'une des revendications 1 à 3, **caractérisé en ce que** la tête de fixation (21) ou respectivement le répartiteur de température (23) est pourvu(e) d'une pluralité d'ouvertures ou de buses de sortie (23).

5. Dispositif selon l'une des revendications 1 à 4, **caractérisé en ce que** le moyen de support et de maintien (17) et/ou la poignée ou le tronçon de maintien est isolé(e) ou comprend au moins des tronçons isolants.

6. Dispositif selon l'une des revendications 1 à 5, **caractérisé en ce qu'**il est prévu au moins une interface détachable (21a, 21b), au moyen de laquelle la tête de fixation (21) et/ou le répartiteur de température (23) peut être relié(e) directement ou au moins indirectement à un système d'admission et en particulier à un tuyau (31).

7. Dispositif selon la revendication 6, **caractérisé en ce que** l'interface détachable (21 a) est prévue entre la tête de fixation (21) et/ou le répartiteur de température (23) et le moyen de maintien et de fixation (17), en particulier la poignée ou le tronçon de maintien (19).

8. Dispositif selon la revendication 6 ou 7, **caractérisé en ce que** l'interface détachable (21b) est prévue soit sur le côté du moyen de maintien et de fixation (17) opposé à la tête de fixation (21) et/ou au répartiteur de température (23), soit dans une région médiane du moyen de maintien et de fixation (17).

9. Dispositif selon l'une des revendications 6 à 8, **caractérisé en ce que** l'interface (21 a, 21b) est une fermeture à vis, à encliquetage, à enclenchement et/ou à baïonnette.

10. Dispositif selon l'une des revendications 1 à 9, **caractérisé en ce qu'**au moins un ou plusieurs systèmes d'actionnement ou leviers d'actionnement (37), pour la commande de l'amenée du milieu ou de l'amenée de vapeur et/ou pour la commande de la température et/ou de la quantité du milieu ou de la vapeur amenée sont prévus sur le moyen de support et de maintien (17), de préférence sur la poignée ou le tronçon de maintien (19) réalisé(e) sur le moyen de support et de maintien (17).

11. Dispositif selon l'une des revendications 1 à 10, **caractérisé en ce que** le moyen de support et de maintien (17) est pourvu d'un système d'actionnement et/ou de levier d'actionnement (37), au moyen duquel le système de commande de température (33) positionné à distance du moyen de support et de maintien (17) et relié via au moins une liaison à tuyau (31) peut être commandé pour un réchauffement et/ou un refroidissement commandé de façon correspondante et/ou pour la commande de l'admission et/ou du dosage de la quantité admise d'un milieu de chauffage ou de refroidissement.

12. Dispositif selon la revendication 11, **caractérisé en ce que** le système de commande de température (33) peut être commandé via un câble non électrique, via une liaison électrique, de préférence une liaison à basse tension et/ou par radio.

13. Dispositif selon l'une des revendications 1 à 12, **caractérisé en ce que** la tête de fixation (21) comprend un logement (45) en forme d'entonnoir.

14. Dispositif selon la revendication 13, **caractérisé en ce qu'**il est prévu des moyens de fixation et/ou de serrage (47) sur la face extérieure du logement (45) en forme d'entonnoir, au moyen desquels des pattes ou des zones de coin (1') réalisées sur l'enveloppement (1) à introduire peuvent être fixées, et ainsi un enveloppement mis en place peut être fixé.

15. Dispositif selon l'une des revendications 1 à 14, **caractérisé en ce qu'**au moins une chambre à produit actif (41) est interposée dans la liaison (31) semblable à un tuyau entre le système de commande de température (33) et la tête de fixation (21), au moyen de laquelle des produits actifs peuvent être admis dans le milieu qui s'écoule en traversant la liaison (31) semblable à un tuyau.

16. Dispositif selon l'une des revendications 1 à 15, **caractérisé en ce que** l'enveloppement (1) peut être relié au moyen de support et de maintien (17) à l'aide d'un système de fixation et/ou de serrage (25).

17. Dispositif selon la revendication 16, **caractérisé en ce que** l'enveloppement comprend une enveloppe extérieure (3), ou peut être amené dans une enveloppe extérieure (9) en forme de poche, dans laquelle la tête de fixation (21) et/ou le répartiteur de température (23) peut être positionné(e).

18. Dispositif selon la revendication 16 ou 17, **caractérisé en ce que** le système de fixation et/ou de serrage (25) comprend des bandes de fixation ou de serrage (27) et/ou une patte de fixation ou de serrage (27'), à la périphérie extérieure de laquelle sont prévus de préférence des tronçons en forme de crochets ou de griffes d'une fermeture agrippante (29) ou similaire, qui coopèrent avec des tronçons d'ancrage ou d'agrippement correspondants sur l'enveloppe extérieure (3) de l'enveloppement (1) ou l'enveloppe extérieure (9) qui reçoit l'enveloppement (1), en vue de la fixation de l'enveloppement (1).

19. Dispositif selon l'une des revendications 1 à 18, **caractérisé en ce que** le système de commande de température (33) comprend un système de chauffage électrique au moyen duquel l'enveloppement (1) peut être chauffé directement ou indirectement.

20. Dispositif selon la revendication 13 ou selon l'une des revendications 14 à 19 en association avec la revendication 13, **caractérisé en ce que** le logement (45) en forme d'entonnoir présente, transversalement à son extension longitudinale, une section circulaire, ovale ou polygonale à n côtés, ou une section transversale qui s'approche de l'une de ces formes.

21. Dispositif selon l'une des revendications 1 à 20, **caractérisé en ce que** le dispositif est pourvu d'un système d'alimentation sous pression pour la mise sous pression d'un enveloppement (1), de préférence sous la forme d'un logement (45) prévu à cet effet, en particulier sous la forme d'un entonnoir.

22. Dispositif selon la revendication 21, **caractérisé en ce que** le système d'alimentation sous pression comprend un mécanisme à vis ou un mécanisme rotatif au moyen duquel un dispositif à pression, de préférence sous la forme d'un répartiteur de température (23), est déplaçable dans le logement (45) en direction de l'enveloppement (1).

23. Dispositif selon la revendication 21 ou 22, **caractérisé en ce que** le logement (45), de préférence en forme d'entonnoir, est au moins en deux parties et comprend un tronçon rotatif, de préférence un tronçon inférieur, au moyen duquel le système d'alimentation sous pression est déplaçable axialement en fonction du mouvement de rotation de la partie, de préférence inférieure, du logement (45).
